(19) Europäisches Patentamt
European Patent Office
Office européen des brevets

(11) Veröffentlichungsnummer : **0 093 959**
**B1**

(12) # EUROPÄISCHE PATENTSCHRIFT

(45) Veröffentlichungstag der Patentschrift : **04.03.87**

(51) Int. Cl.⁴ : **C 07 D405/06**, C 07 D239/70, C 07 D233/06

(21) Anmeldenummer : **83104143.9**

(22) Anmeldetag : **28.04.83**

(54) **Verfahren zur Herstellung von Glycidyl-1,2,4-triazolidin-3,5-dionen.**

(30) Priorität : **12.05.82 DE 3217875**

(43) Veröffentlichungstag der Anmeldung : **16.11.83 Patentblatt 83/46**

(45) Bekanntmachung des Hinweises auf die Patenterteilung : **04.03.87 Patentblatt 87/10**

(84) Benannte Vertragsstaaten : **CH DE FR GB IT LI**

(56) Entgegenhaltungen :
**EP-A- 0 026 312**
**EP-A- 0 051 787**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Rottmaier, Ludwig**
**Bergstrasse 85**
**D-5068 Odenthal (DE)**
Erfinder : **Meyer, Rolf-Volker, Dr.**
**Buchheimerstrasse 23**
**D-4150 Krefeld (DE)**
Erfinder : **Merten, Rudolf, Dr.**
**Berta-von-Suttner-Strasse 55**
**D-5090 Leverkusen 1 (DE)**

**EP 0 093 959 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein verbessertes, zweistufiges Verfahren zur Herstellung an sich bekannter Glycidyl-1,2,4-triazolidin-3,5-dione mit einem bis fünf 1,2,4-Triazolidin-3,5-dionringen und zwei bis zehn, stets an Stickstoff gebundenen Glycidylgruppen pro Molekül. Durch Einsatz cyclischer Amidine als Katalysatoren bei der Umsetzung der 1,2,4-Triazolidin-3,5-dione mit einem Epihalogenhydrin (= Stufe 1) zu den entsprechenden Polyhalogenhydrinen und nachfolgender Dehydrohalogenierung (= Stufe 2) werden die Glycidyl-1,2,4-triazolidin-3,5-dione in höheren Ausbeuten und größerer Reinheit als nach bisher bekannten Verfahren gewonnen. Der Begriff Glycidyl umfaßt Glycidyl (= 2,3-Epoxypropyl) und β-Methylglycidyl (= 2,3-Epoxy-2-methyl-propyl).

Aus der DE-A-2 935 354 (= US-A-4 283 546) ist die Herstellung von 1,2,4-Tri-(β-methyl)-glycidyl-1,2,4-triazolidin-3,5-dionen bekannt. In dem bevorzugten Verfahren dieser Schrift wird in einer ersten Stufe 1,2,4-Triazolidin-3,5-dion mit Epihalogenhydrin oder β-Methylepihalogenhydrin in Gegenwart eines Katalysators zu den Trihalogenhydrinen des 1,2,4-Triazolidin-3,5-dions umgesetzt, und anschließend werden die Halogenhydringruppen mit Halogenwasserstoff abspaltenden Mitteln in einer zweiten Stufe in Epoxidgruppen (= 2,3-Epoxypropyl- und 2,3-Epoxy-2-methyl-propylgruppen) übergeführt. Als Katalysatoren werden u. a. tertiäre Amine sowie quaternäre Ammoniumsalze genannt. Dabei werden 1,2,4-Triglycidyl-1,2,4-triazolidin-3,5-dione erhalten, deren Epoxidwert 12 bis 20 % niedriger als der theoretische Wert liegt. Ferner enthalten alle Produkte, wie aus den Beispielen der zitierten Offenlegungsschrift ersichtlich, über 2 Gew.-% Chlor, was bei bestimmten Anwendungen nicht erwünscht ist. Wird jedoch ein kristallines 1,2,4-Triglycidyl-1,2,4-triazolidin-3,5-dion benötigt, so kann dies nur durch Umkristallisieren der rohen Epoxide erhalten werden.

Nach dem gleichen Verfahren, wie in der DE-A-2 935 354 beschrieben, werden gemäß DE-A-3 027 623 (= US-Anmeldung, Serial N° 278 646) in 4-Stellung durch einen einwertigen Rest $R^1$ substituierte 1,2-Diglycidyl-1,2,4-triazolidin-3,5-dione bzw. in 4-Stellung durch einen zwei- bis fünfwertigen Rest $R^1$ verknüpfte Polyglycidyl-1,2,4-triazolidin-3,5-dione der Formel (A)

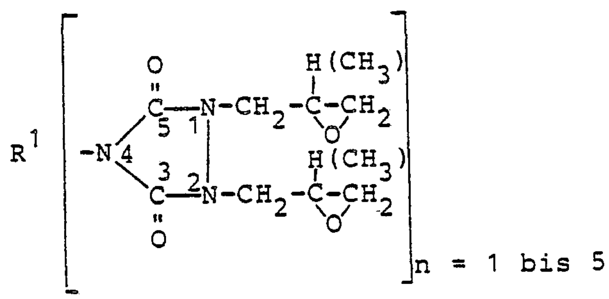

(A)

vorzugsweise erhalten. Auch hier lassen die Reinheit und die Ausbeute der Endprodukte noch Wünsche offen.

In der vorliegenden Erfindung umfaßt der Begriff Glycidyl (= 2,3-Epoxypropyl) auch β-Methylglycidyl (= 2,3-Epoxy-2-methyl-propyl) und der Begriff « ein Epihalogenhydrin » auch die Verbindungen der « β-Methylepihalogenhydrine ». Ferner wird in dieser Erfindung unter « ein 1,2,4-Triazolidin-3,5-dion » oder unter « 1,2,4-Triazolidin-3,5-dione » das 1,2,4-Triazolidin-3,5-dion und die der nachstehenden Formel (B)

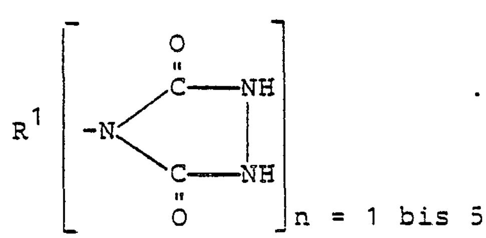

(B)

entsprechenden Verbindungen, in denen $R^1$ einen ein- bis fünfwertigen, gegebenenfalls durch Sauerstoff, tert. Stickstoff oder Sulfonyl unterbrochenen, oder gegebenenfalls durch Alkoxycarbonyl, Alkylmercapto, —CN, —$NO_2$, Dialkylamine und Halogen substituierten aliphatischen, cycloaliphatischen, araliphatischen oder aromatischen Rest,
wie in der DE-A-3 027 623 im einzelnen definiert, verstanden.

Aufgabe der vorliegenden Erfindung war es, die aus den DE-A-2 935 354 und 3 027 623 bekannten Glycidyl-1,2,4-triazolidin-3,5-dione aus einem Epihalogenhydrin und 1,2,4-Triazolidin-3,5-dionen in größerer Reinheit und höherer Ausbeute sowie mit einem niedrigeren Chlorgehalt, als dort beschrieben, herzustellen.

Diese Aufgabe konnte überraschend dadurch gelöst werden, daß bei der Reaktion der 1,2,4-Triazolidin-3,5-dione mit einem Epihalogenhydrin zu den Halogenhydrinen der Triazolidin-3,5-dione als Katalysator mindestens ein cyclisches Amidin verwendet wird.

Gegenstand des vorliegenden Erfindung ist somit ein verbessertes, zweistufiges Verfahren zur Herstellung an sich bekannter Glycidyl-1,2,4-triazolidin-3,5-dione mit einem bis fünf 1,2,4-Triazolidinringen und zwei bis zehn, stets an Stickstoff gebundenen Glycidylgruppen pro Molekül durch Reaktion aus den entsprechenden 1,2,4-Triazolidin-3,5-dionen und 1,2-20 Mol eines Epihalogenhydrins pro NH-Gruppe in Gegenwart mindestens eines Katalysators bei 20-200 °C und anschließender Halogenwasserstoffabspaltung mit mindestens einer alkalisch reagierenden Verbindung bei 10-120 °C in an sich bekannter Weise, dadurch gekennzeichnet, daß als Katalysator mindestens ein cyclisches Amidin der Formel (I)

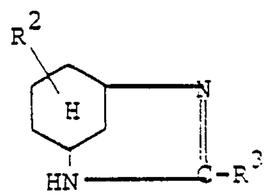

(I)

worin

$R^2$ Wasserstoff oder eine Alkylgruppe mit 1 bis 4 C-Atomen, und

$R^3$ Wasserstoff, eine Alkylgruppe mit 1 bis 18 C-Atomen, eine Cycloalkylgruppe mit 5 bis 14 C-Atomen, eine Aralkylgruppe mit 7 bis 16 C-Atomen oder eine Arylgruppe mit 6 bis 20 C-Atomen bedeuten und/oder ein cyclisches Amidin der Formel (II)

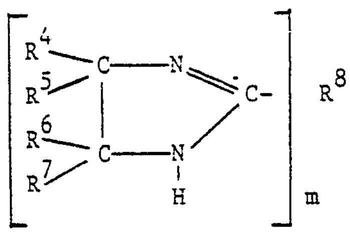

(II)

worin

$R^4$, $R^5$, $R^6$, $R^7$ ein Wasserstoffatom, einen Alkylrest mit 1 bis 6 C-Atomen, einen Cycloalkylrest mit 5 bis 10 C-Atomen, einen Aralkylrest mit 7 bis 12 C-Atomen, einen Arylrest mit 6 bis 15 C-Atomen oder einen heterocyclischen Rest mit 5 bis 10 C-Atomen und mit 1 oder 2 Sauerstoff-, Stickstoff- und/oder Schwefelatomen und

$R^8$ einen Alkylrest mit 1 bis 12 C-Atomen, einen Alkylenrest mit 1 bis 6 C-Atomen, einen Aryl- oder Arylenrest mit 6 bis 15 C-Atomen, die vorstehenden Reste können jeweils durch mindestens einen $C_1$-$C_6$-Alkyl-, $C_5$-$C_{10}$-Cycloalkyl-, $C_7$-$C_{12}$-Aralkyl-, $C_6$-$C_{15}$-Arylrest oder mindestens einen heterocyclischen Rest mit 5 bis 10 C-Atomen und mit 1 oder 2 Sauerstoff-, Stickstoff- und/oder Schwefelatomen substituiert sein, wobei die Substitution des $C_1$-$C_{12}$-Alkyl durch $C_1$-$C_6$-Alkyl ausgeschlossen ist,

m 1 oder 2, und

$R^8$ für den Fall m = 1 auch ein Wasserstoffatom

bedeuten, eingesetzt wird.

Bevorzugte Glycidyl-1,2,4-triazolidin-3,5-dione entsprechen der Formel A, in der $R^1$ Glycidyl und/oder β-Methyl-glycidyl ; Methyl, Butyl, n-Hexyl, 2-Ethylhexyl, Stearyl ; Allyl ; Cyclohexyl ; Phenyl ; Ethylen, Hexamethylen, Trimethylhexamethylen ; Cyclohexylen ; Methylen-bis-cyclohexylen (= $-C_6H_{10}-CH_2-C_6H_{10}-$) ; Ethylendioxy-bis-trimethylen (= $-(H_2C)_3-O(CH_2)_2-O(CH_2)_3-$) und den zweiwertigen Rest des Isophorons der Formel

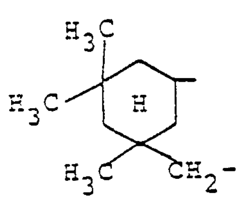

bedeutet.

Als Katalysatoren zu verwendende Amidine sind Imidazolderivate und Tetrahydropyrimidinderivate geeignet.

Vorzugsweise entsprechen die cyclischen Amidin der Formel I

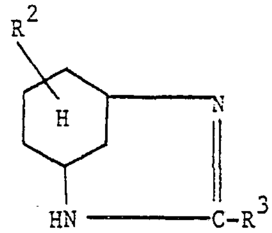
(I)

worin

$R^2$ Wasserstoff oder eine Alkylgruppe mit 1 bis 4 C-Atomen, insbesondere ein Wasserstoffatom oder eine Methylgruppe und

$R^3$ Wasserstoff, eine Alkylgruppe mit 1 bis 18 C-Atomen, eine Cycloalkylgruppe mit 5 bis 14 C-Atomen, eine Aralkylgruppe mit 7 bis 16 C-Atomen oder eine Arylgruppe mit 6 bis 20 C-Atomen, insbesondere Wasserstoff, Methyl, Ethyl, Propyl, 2-Ethylhexyl, Stearyl, Phenyl und Benzyl

bedeuten (vergl. DE-A-3 041 834).

Eine weitere Gruppe bevorzugter cyclischer Amidine entspricht der Formel (II)

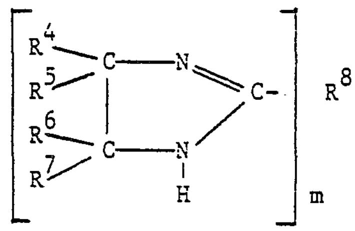
(II)

worin

$R^4$, $R^5$, $R^6$, $R^7$ ein Wasserstoffatom, einen Alkylrest mit 1 bis 6 C-Atomen, einen Cycloalkylrest mit 5 bis 10 C-Atomen, einen Aralkylrest mit 7 bis 12 C-Atomen, einen Arylrest mit 6 bis 15 C-Atomen oder einen heterocyclischen Rest mit 5 bis 10 C-Atomen und mit 1 oder 2 Sauerstoff-, Stickstoff- und/oder Schwefelatomen und

$R^8$ einen Alkylrest mit 1 bis 12 C-Atomen, einen Alkylenrest mit 1 bis 6 C-Atomen, einen Aryl- oder Arylenrest mit 6 bis 15 C-Atomen, die vorstehenden Reste können jeweils durch mindestens einen $C_1$-$C_6$-Alkyl-, $C_5$-$C_{20}$-Cycloalkyl-, $C_7$-$C_{19}$-Aralkyl-, $C_6$-$C_{15}$-Arylrest oder mindestens einen heterocyclischen Rest mit 5 bis 10 C-Atomen und mit 1 oder 2 Sauerstoff-, Stickstoff- und/oder Schwefelatomen substituiert sein, wobei die Substitution des $C_1$-$C_{12}$-Alkylrestes durch $C_1$-$C_6$-Alkyl ausgeschlossen ist.

m 1 oder 2 und

$R^8$ für den Fall m = 1 auch ein Wasserstoffatom

bedeuten (vergl. DE-A-3 041 834).

Namentlich seien die folgenden cyclischen Amidine der Formel (II) genannt : 2-Phenylimidazolin, 2-Phenyl-4-methylimidazolidin, 2-(m-Tolyl)-4-methyl-imidazolin, 2-(m-Pyridyl)-imidazolin, 1,4-Tetramethylen-bis-(4-methyl-imidazolin-2), 2-Methyl-imidazolin, 2,4-Dimethyl-imidazolin, 2-Ethyl-imidazolin, 2-Ethyl-4-methyl-imidazolin, 2-Benzyl-imidazolin, 2-(o-Tolyl)-imidazolin, 2-(p-Tolyl)-imidazolin, Tetra-methylen-bis-imidazolin, 1,1,3-Trimethyl-1,4-tetramethylen-bis-imidazolin, 1,1,3-Trimethyl-1,4-tetramethylen-bis-4-methyl-imidazolin, 1,3,3-Trimethyl-1,4-tetramethylen-bis-methyl-imidazolin, 1,2-Phenylen-bis-imidazolin, 1,3-Phenylen-bis-4-methyl-imidazolin. Es können auch Gemische der Imidazolin-Derivate eingesetzt werden ; besonders bevorzugt werden 2-Phenyl-imidazolin oder 2-Methyl-imidazolin.

Die erfindungsgemäß zu verwendenden Amidine der allgemeinen Formel (I) lassen sich in bekannter Weise durch Reaktion der cycloaliphatischen Diamine der allgemeinen Formel (III) mit Monocarbonsäuren oder deren reaktionsfähigen Derivate der allgemeinen Formel (IV)

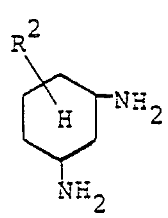
(III) ; $R^3 - R^9$ (IV)

worin

$R^2$ und $R^3$ die oben für Formel (I) angegebene Bedeutung haben und

$R^9$ —COCl, —CN, —$CO_2R^{10}$ ist mit
$R^{10}$ = Wasserstoffatom, $C_1$-$C_6$-Alkyl, Phenyl oder Benzyl, herstellen.

Neben 1,3-Diaminocyclohexan selbst sind bevorzugte Verbindungen (III) insbesondere die Methyl-1,3-diaminocyclohexane, die durch Hydrierung der großtechnisch anfallenden Toluylendiamine entstehen. Die 2,4- und die 2,6-Diaminisomeren sowie ihre Gemische lassen sich gleichermaßen gut verwenden.

Bevorzugte Carbonsäuren (IV) sind Ameisensäure, Essigsäure, Propionsäure, Buttersäure, 2-Ethylhexansäure, Stearinsäure, Benzoesäure und Phenylessigsäure.

Die cyclischen Amidine der Formel (I) werden durch Umsetzung von 1 Mol Carbonsäure oder Carbonsäurederivat der Formel (IV) mit 1 bis 2,5, vorzugsweise 1 bis 1,5 Mol Diamin der Formel (III) bei 150-280 °C, vorzugsweise 200 bis 250 °C innerhalb von 2 bis 10 Stunden erhalten.

Die erfindungsgemäß einsetzbaren cyclischen Amidine der Formel (II) sind bekannt (siehe DE-A-3 041 834). Sie können nach bekannten Verfahren aus gegebenenfalls substituierten geminalen Derivaten und aliphatischen oder aromatischen Mono- bzw. Dinitrilen, gegebenenfalls in Gegenwart von $H_2S$, elementarem Schwefel oder Sulfurylchlorid als Katalysator hergestellt werden.

Die cyclischen Amidine der Formeln (I) und (II), von denen die der Formel (I) bevorzugt sind, können sowohl in reiner Form als auch in Form der technisch zuerst entstehenden Rohprodukte, das heißt ohne weitere Reinigung, als Katalysatoren eingesetzt werden.

Die Menge an verwendetem Katalysator (cyclisches Amidin) liegt vorzugsweise zwischen 0,1 und 10 Mol-%, bezogen auf eingesetzt 1,2,4-Triazolidin-3,5-dione.

Die Reaktion der 1,2,4-Triazolidin-3,5-dione mit einem Epihalogenhydrin, vorzugsweise Epichlorhydrin bzw. β-Methylepichlorhydrin, wird mit mindestens äquivalenten Mengen an einem Epihalogenhydrin durchgeführt, d. h. eine NH-Gruppe der 1,2,4-Triazolidin-3,5-dione wird mit mindestens einem Mol Epihalogenhydrin zur Reaktion gebracht. Vorzugsweise wird jedoch ein Überschuß an Epihalogenhydrin, und zwar 1,2 bis 20 Mol, besonders bevorzugt 3 bis 10 Mol Epihalogenhydrin pro NH-Gruppe, verwendet. Aus wirtschaftlichen Gründen wird man die Menge an Epihalogenhydrin natürlich so niedrig wie möglich halten.

Die Reaktion zwischen den 1,2,4-Triazolidin-3,5-dionen und einem Epihalogenhydrin wird bei 20 bis 200 °C, vorzugsweise bei 60 bis 100 °C, gegebenenfalls unter erhöhtem Druck, durchgeführt.

Die Reaktionszeiten liegen im allgemeinen zwischen 30 Minuten und mehreren Tagen, können in besonderen Fällen jedoch auch darunter liegen. Durch entsprechende Wahl der Reaktionsbedingungen, z. B. Druck, werden kürzere Reaktionszeiten erreicht.

Anschließend wird in einer zweiten Stufe die Halogenhydrinverbindung, die jedoch je nach Epihalogenhydrin- und/oder β-Methylhalogenhydrinüberschuß bereits gewisse Mengen an Glycidylverbindungen enthalten kann, mittels Halogenwasserstoff-abspaltender Verbindungen zu den Glycidyl-1,2,4-triazolidin-3,5-dionen der Formel (A) bzw. zum 1,2,4-Triglycidyl-1,2,4-triazolidin-3,5-dion dehydrohalogeniert.

Die zur Halogenwasserstoffabspaltung eingesetzten alkalisch reagierenden Verbindungen sind insbesonders Alkali- oder Erdalkalimetallhydroxide wie Natriumhydroxid, Kaliumhydroxid, Calciumhydroxid, vorzugsweise Natriumhydroxid. Dies kann in fester Form oder in wäßriger Lösung, vorzugsweise in 20-50 %igen Lösungen, eingesetzt werden.

Ferner können zur Halogenwasserstoffabspaltung Alkalicarbonate, insbesondere Soda und Kaliumcarbonat in fester oder gelöster Form, Alkalisilikate, Alkaliphosphate und Alkalialuminate sowie überschüssiges Epihalogenhydrin, oder 1,2-Alkylenoxide, wie Ethylenoxid Verwendung finden, wobei bei Verwendung von Epichlorhydrin dieses in Glycerindichlorhydrin umgewandelt wird.

Auf eine Halogenhydrin-Gruppe der 1,2,4-Triazolidin-3,5-dione werden 1 bis 1,2 Äquivalente der Halogenwasserstoff abspaltenden Verbindungen eingesetzt.

Zweckmäßig soll bei der Halogenwasserstoffabspaltung der pH-Wert nicht über 13, vorzugsweise nicht über pH 11 ansteigen. Um dies zu erreichen, setzt man das Alkali nach und nach zu bzw. tropf die Lösung allmählich zu und kontrolliert dabei den pH-Wert der Reaktionsmischung.

Die Halogenwasserstoffabspaltung kann im Temperaturbereich von 10 °C bis 120 °C erfolgen. Wird die Halogenwasserstoffabspaltung mit Alkali, z. B. Kalilauge, durchgeführt, so ist zur Erzielung optimaler Ausbeuten eine Reaktionstemperatur von 70 °C nicht zu überschreiten. Die besten Ergebnisse werden bei Temperaturen um 25 bis 35 °C erhalten. Bei Einsatz von Alkalicarbonaten sollte die Reaktionstemperatur jedoch über 70 °C liegen, wobei die Höchstgrenze in der Regel durch die Siedetemperatur des überschüssigen Epihalogenhydrins vorgegeben ist.

Bei der Dehydrohalogenierung ist es vorteilhaft, ein mit Wasser praktisch nicht mischbares organisches Lösungsmittel zuzusetzen, um das bei der Reaktion entstehende bzw. das über die Alkalilösung zugetropfte Wasser azeotrop entfernen zu können. Die Mengen an zugesetztem Lösungsmittel sind nicht kritisch. Geeignete Lösungsmittel sind chlorierte Kohlenwasserstoffe wie Methylenchlorid, Chloroform, Ethylenchlorid oder Trichlorethylen. Sofern für die Bildung der 1,2,4-Triazolidin-3,5-dionchlorhydrinverbindungen ein großer Überschuß an Epihalogenhydrin eingesetzt wurde, kann das überschüssige Epihalogenhydrin als mit Wasser nicht mischbares Lösungsmittel fungieren.

Die Aufarbeitung des 1,2,4-Glycidyl-1,2,4-triazolidin-3,5-dions und der Glycidyl-1,2,4-triazolidin-3,5-dione der Formel (A) erfolgt in der Regel so, daß das bei der Halogenwasserstoffabspaltung entstehende Nebenprodukt, z. B. Kochsalz bei Verwendung von Natronlauge als Säurefänger, durch Absaugen

entfernt wird. Eventuell noch vorhandene Kochsalz- und Alkalireste werden durch Auswaschen mit Wasser entfernt. Selbstverständlich können jedoch auch das gesamte Kochsalz und eventuell vorhandene Alkalireste ohne vorheriges Absaugen durch Auswaschen mit Wasser entfernt werden. Die verbleibende Lösung wird dann gegebenenfalls nach dem Trocknen über einem geeigneten Trocknungsmittel wie wasserfreiem Natriumsulfat vom Lösungsmittel, z. B. überschüssigem Epihalogenhydrin, welches in den nächsten Ansätzen wieder mitverwendet werden kann, gegebenenfalls im Vakuum entfernt, und das erhaltene hellgelb bis gelb gefärbte viskose Öl wird gegebenenfalls durch Auflösen in geeigneten Lösungsmitteln wie $C_1$-$C_4$-Alkanolen, vorzugsweise Methanol, Ethanol, n-Butanol, Ketonen wie Butanon, Glykol- bzw. Diglykolmonoether oder deren Acetate wie Ethylenglykolmonomethylether, Diethylenglykolmonoethylether, Ethylenglykolmonomethyletheracetat und Carbonsäureester wie Essigsäureethylester und nachfolgendem Abkühlen zur Kristallisation gebracht.

Die erhaltenen, kristallinen Verbindungen, können abgesaugt und gegebenenfalls durch Umkristallisieren, z. B. aus Methanol, weiter gereinigt werden. Häufig kann jedoch auf eine Reinigung verzichtet und das Rohprodukt sofort weiterverarbeitet werden. Je nach Konstitution des Restes $R^1$ entstehen häufig jedoch dünnflüssige bis hochviskose Harze, die dann ohne weitere Reinigung weiter umgesetzt werden können.

Die nach dem erfindungemäßen Verfahren hergestellten Glycidyl-1,2,4-triazolidin-3,5-dione besitzen Epoxidwerte von 0,2 bis 1,13, vorzugsweise 0,6 bis 1,13. Unter Epoxidwert wird die Anzahl von Epoxidäquivalenten verstanden, die in 100 g Substanz enthalten sind. Das Epoxidäquivalent ist definiert als die Grammenge Substanz, in der eine 1,2-Epoxidgruppe enthalten ist. Eine 1,2-Epoxidgruppe ist einem Mol Halogenwasserstoff äquivalent. Von der Herstellung her können die Polyglycidylverbindungen der 1,2,4-Triazolidin-3,5-dione noch verseifbares Halogen (bis ca. 2,5 Gew.-% Chlor bzw. ca. 6,5 Gew.-% Brom, vorzugsweise bis ca. 1,5 Gew.-% Chlor bzw. ca. 4 Gew.-% Brom) enthalten, das durch weitere Behandlung mit Halogenwasserstoff abspaltenden Mitteln, falls gewünscht, praktisch vollständig entfernt werden kann und zumindest teilweise den Gehalt an Epoxidgruppen erhöht.

Überraschenderweise hat sich gezeigt, daß durch die Verwendung der cyclischen Amidine als Katalysatoren schon als Rohprodukte Glycidyl-1,2,4-triazolidin-3,5-dione erhalten werden, welche einen wesentlich niedrigeren Chlorgehalt aufweisen als die Glycidyl-1,2,4-triazolidin-3,5-dione, welche mit bekannten Katalysatoren wie tertiären Aminen und quaternären Ammoniumsalzen hergestellt werden. Weiterhin werden durch die Verwendung der cyclischen Amidine Glycidyl-1,2,4-triazolidin-3,5-dione erhalten, deren Epoxidwerte schon in den Rohprodukten merklich höher liegen als die der Glycidyl-1,2,4-triazolidin-3,5-dione, welche bei der Synthese mit den bekannten Katalysatoren erhalten werden. Dies führt bei verschiedenen Produkte dazu, daß erst durch den Einsatz der cyclischen Amidine solch reine Glycidyl-1;2,4-triazolidin-3,5-dione entstehen, daß diese kristallisiert werden können. Auch liegen die Ausbeuten an Rohprodukten der erfindungsgemäß erhaltenen Glycidyl-1,2,4-triazolidin-3,5-dione in den meisten Fällen merklich höher als nach den bisher bekannter Verfahren.

Die nach dem erfindungsgemäßen Verfahren erhaltenen Glycidyl-1,2,4-triazolidin-3,5-dione können allein oder zusammen mit üblichen Härtungsmitteln als Imprägnierungsmittel für Textilien, z. B. Fasern aus Polyester, als Überzugsmittel, z. B. für Anstrichzwecke auf Glas, Metallen und Holz, als Klebemittel für Kunststoffe verschiedenster Art, z. B. zum Verkleben von Formkörpern, z. B. Gieß-Preßkörpern und Laminaten verwendet werden. So kann das 1,2,4-Triglycidyl-1,2,4-triazolidin-3,5-dion als Vernetzer für Carboxylgruppen-haltige Polymere, wie in der DE-A-2 935 446 (= US-A-4 288 569) beschrieben, nach den elektrostatischen Pulversprühverfahren zur Herstellung von temperaturbeständigen Beschichtungen verwendet werden.

Die nachfolgenden Beispiele sollen die Erfindung näher erläutern, wobei, wenn nicht anders angegeben, die Prozente und Teile sich auf das Gewicht beziehen.

Herstellung eines Amidins der Formel I mit $R^2$ = Methyl und $R^3$ = Phenyl (Isomerengemisch)

In einem 4 l Dreihalskolben, versehen mit Rührer, Thermometer und Claisenbrücke mit einem 1 l Vorlagekolben, werden 768 g (6 Mol) eines Isomerengemisches, bestehend aus ca. 80 % 2,4- und 20 % 2,6-Diamino(methyl-cyclohexan), in folgenden « PH-Tolamin » genannt, und 200 ml Wasser unter Rühren und $N_2$-Schutzgas vorgelegt. Dazu werden 732 g (6 Mol) Benzoesäure portionsweise innerhalb von 30 Minuten eingetragen. Die Temperatur steigt dabei auf 80 °C an. Sodann wird zügig auf 250 °C aufgeheizt, wobei Wasser und überschüssiges PH-Tolamin abdestillieren. Nach 4 Stunden bei 250 °C wird die Reaktion beendet. Das Destillat enthält neben Wasser 250 g PH-Tolamin. Das verbleibende Reaktionsprodukt wird bei ca. 0,3 mbar destilliert (bis 240 °C). Man erhält 733 g Rohprodukt, das zu gelblichen Kristallen erstarrt und 346 g Rückstand. Die Destillation des Rohproduktes ergibt folgende Fraktion :

Vorlauf $Kp_{0,3\ mbar}$ 80-160 °C = 35 g (ca. 90 % PH-Tolamin)

Hauptlauf $Kp_{0,3\ mbar}$ 160-195 °C = 671 g Amidin, weiße Kristalle Fp 50-60 °C, Rückstand : 27 g

Die Konstitution des Amidins wurde durch IR- und NMR-Spektren sowie durch Elementaranalyse bestätigt.

Analog der vorstehenden Vorschrift werden die anderen Amidine der Formel I hergestellt.

## Beispiel 1

460 g 4-Methyl-1,2,4-triazolidin-3,5-dion, 3 600 g Epichlorhydrin und 8 g Amidin der allgemeinen Formel I mit $R^2$ = Methyl und $R^3$ = Phenyl werden auf 60 °C erwärmt und 22 Stunden bei 60 °C gerührt. Zu der abgekühlten klaren Lösung werden bei 25 bis 30 °C 328 g schuppenförmiges Natriumhydroxid in kleinen Portionen innerhalb von 2,5 Stunden zugegeben. Zur Vervollständigung der Reaktion wird 5 Stunden bei 30 °C nachgerührt. Nach dem Absaugen des Kochsalzes wird die Epichlorhydrinlösung mit 200 g Wasser ausgewaschen, über Natriumsulfat getrocknet und im Vakuum bei 60 °C, zuletzt bei 0,3 mbar eingeengt. Es verbleiben 812 g eines leicht gelblich gefärbten Epoxidharzes mit einem Epoxidwert von 0,83 und einem Chlorgehalt von 0,6 % Gesamtchlor, welches beim Abkühlen auf Raumtemperatur erstarrt. Durch Umkristallisieren mit 200 ml Ethanol werden aus 100 g des rohen Epoxids 78 g praktisch reines 1,2-Bisglycidyl-4-methyl-1,2,4-triazolidin-3,5-dion von Fp = 114-116 °C erhalten.

Vergleichsbeispiel 1 (gemäß den Bedingungen der DE-A-3 027 623)

460 g 4-Methyl-1,2,4-triazolidin-3,5-dion, 3 600 g Epichlorhydrin und 8 g Triethylamin werden analog Beispiel 1 zum Epoxid umgesetzt. Es werden 768 g eines gelb gefärbten Epoxidharzes mit einem Epoxidwert von 0,75 und einem Chlorgehalt von 1,8 % Gesamtchlor, welches beim Stehen über mehrere Tage zu einer schmierigen Masse durchkristallisiert. Durch Umkristallisieren mit 200 ml Ethanol werden aus 100 g des rohen Epoxids 52 g praktisch reines 1,2-Bisglycidyl-4-methyl-1,2,4-triazolidin-3,5-dion von Fp = 113-115 °C erhalten.

Beispiel 2

183 g 4-Cyclohexyl-1,2,4-triazolidin-3,5-dion, 1 400 g Epichlorhydrin und 2 g Amidin der allgemeinen Formel I mit $R^2$ = Methyl und $R^3$ = Phenyl werden auf 60 °C erwärmt und 18 Stunden bei 60 °C gerührt. Nach dem Abkühlen werden bei 25 bis 30 °C innerhalb 2 Stunden 82 g geschupptes Natriumhydroxid in 6 Portionen zugegeben. Zur Vervollständigung der Reaktion wird 4 Stunden bei 30 °C nachgerührt. Das entstandene Kochsalz wird abfiltriert und die Epichlorhydrinlösung mit 100 g Wasser gewaschen, über Natriumsulfat getrocknet und im Vakuum eingeengt. Der Rückstand wird bei 70 °C und 0,25 mbar bis zur Gewichtskonstanz getrocknet. Es verbleiben 265 g eines bernsteinfarbenen viskosen Epoxidharzes mit einem Epoxidwert von 0,61 und einem Chlorgehalt von 0,9 % Gesamtchlor, welches nach eintägigem Stehen durchzukristallisieren beginnt. Durch Verreiben mit n-Butanol/Cyclohexan (2 : 1) wird praktisch reines 1,2-Bisglycidyl-4-cyclohexyl-1,2,4-triazolidin-3,5-dion von Ep = 98-99 °C (aus n-Butanol/Cyclohexan 1 : 1) erhalten. IR- und NMR-Spektren sowie die Elementaranalyse bestätigen die angenommene Struktur.

Elementaranalyse berechnet für $C_{14}H_{21}N_3O_4$ (Molekulargewicht 295,3).
berechnet : C = 56,93 % ; H = 7,17 % ; N = 14,23 %
gefunden : C = 56,8 % ; H = 7,1 % ; N = 14,1 %

Vergleichsbeispiel 2 (gemäß den Bedingungen der DE-A-3 027 623)

183 g 4-Cyclohexyl-1,2,4-triazolidin-3,5-dion, 1 400 g Epichlorhydrin und 2 g Tetramethylammoniumchlorid werden analog Beispiel 2 umgesetzt und aufgearbeitet. Es werden 280 g eines gelben, viskosen Epoxidharzes mit einem Epoxidwert von 0,52 und einem Chlorgehalt von 2,1 % Gesamtchlor erhalten, welches auch nach 4-monatigem Stehen nicht kristallisiert.

Beispiel 3

202 g 1,2,4-Triazolidin-3,5-dion, 1 400 g Epichlorhydrin und 4 g Amidin der allgemeinen Formel I mit $R^2$ = Methyl und $R^3$ = Phenyl werden auf 60 °C erwärmt und 24 Stunden bei 60 °C gerührt. Zu der abgekühlten klaren Lösung werden bei 25 bis 30 °C innerhalb von 4 Stunden 246 g geschupptes NaOH in kleinen Portionen zugesetzt. Zur Vervollständigung der Reaktion wird 4 Stunden bei 30 °C nachgerührt. Sodann wird vom unlöslichen Rückstand filtriert, die Epichlorhydrinlösung mit 100 g Wasser ausgewaschen, über Natriumsulfat getrocknet und im Vakuum eingeengt. Der Rückstand wird bei 70 °C/0,25 mbar bis zur Gewichtskonstanz getrocknet. Es werden 448 g einer bernsteinfarbigen öligen Flüssigkeit mit einem Epoxidwert von 1,06 und einem Chlorgehalt von 0,7 % Gesamtchlor erhalten, die beim Abkühlen zu einer harten Masse erstarrt und die laut Gelchromatographie zu 93 % aus 1,2,4-Triglycidyl-triazolidin-3,5-dion besteht (Rest sind höhermolekulare Epoxide). Durch Umkristallisieren aus Methanol oder Ethanol oder Essigsäureethylester kann ein fast reines Triepoxid (Reinheit lt. Gelchromatographie > 99 %) vom Fp = 97-104 °C erhalten werden.

Vergleichsbeispiel 3 (gemäß den Bedingungen der DE-A-2 935 354)

202 g 1,2,4-Triazolidin-3,5-dion, 1 400 g Epichlorhydrin und 4 g Triethylamin werden analog Beispiel 3 umgesetzt und aufgearbeitet. Es werden 388 g einer gelben, öligen Flüssigkeit mit einem Epoxidwert von 0,98 und einem Chlorgehalt von 1,7 % Gesamtchlor erhalten, die über Nacht zu einer schmierigen

Masse durchkristallisiert. Dieses rohe Epoxid kann nicht zur Herstellung von Pulverlacken, wie in der DE-OS-2 935 446 beschrieben, verwendet werden, während das rohe Epoxid aus Beispiel 3 dafür geeignet ist.

Beispiel 4

202 g 1,2,4-Triazolidin-3,5-dion, 1 400 g Epichlorhydrin und 4 g Amidin der Formel I mit $R^2$ = Methyl und $R^3$ = Stearyl werden 60 Stunden bei 60 °C gerührt. Die klare Reaktionslösung wird wie in Beispiel 3 weiter aufgearbeitet. Es werden nach dem Abestillieren der flüchtigen Bestandteile 432 g einer gelblichen, öligen Flüssigkeit mit einem Epoxidwert von 1,02 und einem Chlorgehalt von 0,9 % Gesamtchlor erhalten, die beim Abkühlen zu einer harten Masse erstarrt. Laut Gelchromatographie ist der Reinheitsgrad des 1,2,4-Triglycidyl-1,2,4-triazolidin-3,5-dions ca. 92,5 %.

Beispiel 5

Beispiel 4 wird wiederholt, jedoch werden als Katalysator 4 g des Amidins der Formel I mit $R^2$ und $R^3$ = Methyl eingesetzt (Reaktionszeit bei der Chlorhydrinbildung = 30 Stunden). Es werden 439 g einer gelblichen, öligen Flüssigkeit mit einem Epoxidwert von 1,05 und einem Chlorgehalt von 0,7 % Gesamtchlor erhalten, die beim Abkühlen zu einer harten Masse erstarrt. Reinheitsgrad des 1,2,4-Triglycidyl-1,2,4-triazolidin-3,5-dions ca. 93 %.

Beispiel 6

Beispiel 4 wird wiederholt, jedoch werden als Katalysator 4 g des Amidins der Formel I mit $R^2$ = Methyl und $R^3$ = Wasserstoff eingesetzt (Reaktionszeit bei der Chlorhydrinbildung = 18 Stunden). Es werden 454 g einer leicht gelblichen, öligen Flüssigkeit mit einem Epoxidwert von 1,05 und einem Chlorgehalt von 1,0 % Gesamtchlor erhalten, die beim Abkühlen zu einer harten Masse erstarrt. Reinheitsgrad des 1,2,4-Triglycidyl-1,2,4-triazolidin-3,5-dions laut Gelchromatographie ca. 94 %.

Beispiel 7

202 g 1,2,4-Trizolidin-3,5-dion, 1 400 g Epichlorhydrin und 4 g Phenylimidazolin (Formel II mit $R^4$, $R^5$, $R^6$, $R^7$ für Wasserstoff, $R^8$ für Phenyl und n für 1) werden 23 Stunden bei 60 °C gerührt. Die klare Lösung wird wie in Beispiel 3 weiter aufgearbeitet. Es werden 435 g einer gelblichen, öligen Flüssigkeit mit einem Epoxidwert von 1,03 und einem Chlorgehalt von 1,1 % Gesamtchlor erhalten, die beim Abkühlen zu einer harten Masse erstarrt. Reinheitsgrad des 1,2,4-Triglycidyl-1,2,4-triazolidin-3,5-dions ca. 92 % laut Gelchromatographie.

Beispiel 8

142 g 1,6-Hexandiyl-4,4'-bis-1,2,4-triazolidin-3,5-dion, 1 200 g Epichlorhydrin und 1 g Amidin der Formel I mit $R^2$ = Methyl und $R^3$ = Phenyl werden 30 Stunden bei 60 °C gerührt. Nach dem Abkühlen werden 82 g geschupptes Natriumhydroxid bei 25 bis 30 °C in kleinen Portionen in 2 Stunden zugegeben. Zur Vervollständigung der Reaktion wird noch 5 Stunden bei 30 °C nachgerührt. Das entstandene Kochsalz wird abgesaugt, die Epichlorhydrinlösung mit 100 g Wasser ausgewaschen, über Natriumsulfat getrocknet und im Vakuum eingeengt. Nach dem Trocknen bei 60 °C und 0,3 mbar bis zur Gewichtskonstanz werden 242 g eines gelblich gefärbten Epoxidharzes mit einem Epoxidwert von 0,76 und einem Chlorgehalt von 0,9 % Gesamtchlor erhalten.

Nach dem Verfahren des Beispiels 4 der DE-OS-3 027 623 besitzt das Tetraepoxid des 1,6-Hexandiyl-4,4'-bis-1,2,4-triazolidin-3,5-dions einen Epoxidwert von 0,73 und einen Chlorgehalt von 1,6 % Gesamtchlor.

Beispiel 9

28,2 g 4-Allyl-1,2,4-triazolidin-3,5-dion, 250 g Epichlorhydrin und 0,4 g Amidin der Formel I mit $R^2$ = Methyl und $R^3$ = Phenyl werden 16 Stunden bei 60 °C gerührt. Nach dem Abkühlen werden 23 g Kaliumhydroxid als 40 %ige Lösung bei 30 °C in einer Stunde zugetropft. Zur Vervollständigung der Reaktion wird 4 Stunden bei 30 °C nachgerührt und die anorganischen Bestandteile mit 50 g Wasser ausgewaschen. Die Epichlorhydrinlösung wird über Natriumsulfat getrocknet und im Vakuum eingeengt. Nach dem Trocknen bei 60 °C und 0,25 mbar bis zur Gewichtskonstanz werden 46 g eines hellgelben Epoxidharzes mit einem Epoxidwert von 0,75 und einem Chlorgehalt von 0,8 % Gesamtchlor erhalten, das beim Abkühlen durchkristallisiert. Nach dem Umkristallisieren in Ethanol wird das fast reine 1,2-Bisglycidyl-4-allyl-1,2,4-triazolidin-3,5-dion von Fp = 87-88 °C in Form von weißen Kristallen erhalten. IR- und NMR-Spektren sowie die Elementaranalyse beweisen die angenommene Struktur.

Elementaranalyse für $C_{11}H_{15}N_3O_4$ ; Molekulargewicht 253,3 :

berechnet : C = 52,17 % ; H = 5,97 % ; N = 16,59 %

gefunden : C = 52,1 % ; H = 6,1 % ; N = 16,6 %

**Patentanspruch**

Ein verbessertes, zweistufiges Verfahren zur Herstellung an sich bekannter Glycidyl-1,2,4-triazolidin-3,5-dione mit einem bis fünf 1,2,4-Triazolidinringen und zwei bis zehn, stets an Stickstoff gebundenen Glycidylgruppen pro Molekül durch Reaktion aus den entsprechenden 1,2,4-Triazolidin-3,5-dionen und 1,2-20 Mol eines Eiphalogenhydrins pro NH-Gruppe in Gegenwart mindestens eines Katalysators bei 20-200 °C und anschließender Halogenwasserstoffabspaltung mit mindestens einer alkalisch reagierenden Verbindung bei 10-120 °C, dadurch gekennzeichnet, daß als Katalysator mindestens ein cyclisches Amidin der Formel (I)

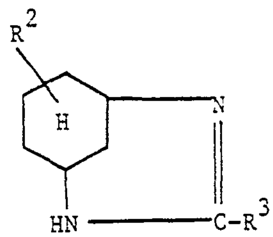

(I)

worin

$R^2$ Wasserstoff oder eine Alkylgruppe mit 1 bis 4 C-Atomen, und

$R^3$ Wasserstoff, eine Alkylgruppe mit 1 bis 18 C-Atomen, eine Cycloalkylgruppe mit 5 bis 14 C-Atomen, eine Aralkylgruppe mit 7 bis 16 C-Atomen oder eine Arylgruppe mit 6 bis 20 C-Atomen bedeuten und/oder ein cyclisches Amidin der Formel (II)

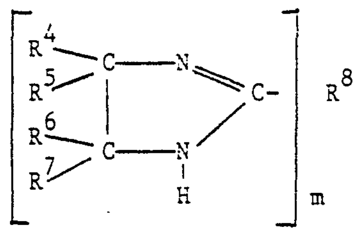

(II)

worin

$R^4$, $R^5$, $R^6$, $R^7$ ein Wasserstoffatom, einen Alkylrest mit 1 bis 6 C-Atomen, einen Cycloalkylrest mit 5 bis 10 C-Atomen, einen Aralkylrest mit 7 bis 12 C-Atomen, einen Arylrest mit 6 bis 15 C-Atomen oder einen heterocyclischen Rest mit 5 bis 10 C-Atomen und mit 1 oder 2 Sauerstoff-, Stickstoff- und/oder Schwefelatomen und

$R^8$ einen Alkylrest mit 1 bis 12 C-Atomen, einen Alkylenrest mit 1 bis 6 C-Atomen, einen Aryl- oder Arylenrest mit 6 bis 15 C-Atomen, die vorstehenden Reste können jeweils durch mindestens einen $C_1$-$C_6$-Alkyl-, $C_5$-$C_{10}$-Cycloalkyl-, $C_7$-$C_{12}$-Aralkyl-, $C_6$-$C_{15}$-Arylrest oder mindestens einen heterocyclischen Rest mit 5 bis 10 C-Atomen und mit 1 oder 2 Sauerstoff-, Stickstoff- und/oder Schwefelatomen substituiert sein, wobei die Substitution des $C_1$-$C_{12}$-Alkyl durch $C_1$-$C_6$-Alkyl ausgeschlossen ist,

m 1 oder 2, und

$R^8$ für den Fall m = 1 auch ein Wasserstoffatom

bedeuten, eingesetzt wird.

**Claim**

An improved two-stage process for the preparation of glycidyl-1,2,4-triazolidine-3,5-diones which contain, per molecule one to five 1,2,4-triazolidine rings and two to ten glycidyl groups always bonded to nitrogen, and which are known *per se*, by reaction from the corresponding 1,2,4-triazolidine-3,5-diones and 1,2-20 mols of an epihalogenhydrin per NH group in the presence of at least one catalyst at 20-200 °C and subsequent cleavage of hydrogen halide with at least one alkaline-reacting compound at 10-120 °C, characterised in that at least one cyclic amidine of the formula (I)

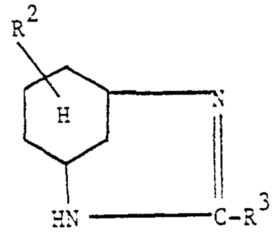

(I)

wherein

$R^2$ denotes hydrogen or an alkyl group with 1 to 4 C atoms and

$R^3$ denotes hydrogen, an alkyl group with 1 to 18 C atoms, a cycloalkyl group with 5 to 14 C atoms, an aralkyl group with 7 to 16 C atoms or an aryl group with 6 to 20 C atoms,

and/or a cyclic amidine of the formula (II)

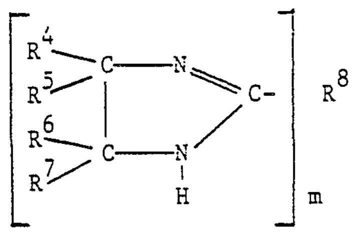

(II)

wherein

$R^4$, $R^5$, $R^6$ and $R^7$ denote a hydrogen atom, an alkyl radical with 1 to 6 C atoms, a cycloalkyl radical with 5 to 10 C atoms, an aralkyl radical with 7 to 12 C atoms, an aryl radical with 6 to 15 C atoms or a heterocyclic radical with 5 to 10 C atoms and with 1 or 2 oxygen, nitrogen and/or sulphur atoms and

$R^8$ denotes an alkyl radical with 1 to 12 C atoms, an alkylene radical with 1 to 6 C atoms, or an aryl or arylene radical with 6 to 15 C atoms, the foregoing radicals can each be substituted by at least one $C_1$-$C_6$-alkyl, $C_5$-$C_{10}$-cycloalkyl, $C_7$-$C_{12}$-aralkyl, or $C_6$-$C_{15}$-aryl radical or at least one heterocyclic radical with 5 to 10 C atoms and with 1 or 2 oxygen, nitrogen and/or sulphur atoms, the substitution of $C_1$-$C_{12}$-alkyl by $C_1$-$C_6$-alkyl being excluded,

m denotes 1 or 2, and

$R^8$, if m = 1, also denotes a hydrogen atom,

is used as the catalyst.

**Revendication**

Procédé perfectionné en deux étapes pour préparer des glycidyl-1,2,4-triazolidine-3,5-diones, connues en soi, comportant par molécule 1 à 5 noyaux 1,2,4-triazolidine et 2 à 10 groupes glycidyle toujours fixés sur l'azote, par réaction des 1,2,4-triazolidine-3,5-diones correspondantes et de 1,2 à 20 moles d'une épihalogénhydrine par groupe NH en présence d'au moins un catalyseur à 20-200 °C puis enlèvement d'un hydracide halogéné à l'aide d'au moins un composé à réaction alcaline à 10-120 °C, procédé caractérisé en ce qu'on utilise comme catalyseur au moins une amidine cyclique de formule (I)

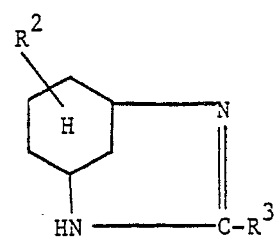

(I)

dans laquelle :

$R^2$ représente un atome d'hydrogène ou un groupe alkyle ayant 1 à 4 atomes de carbone, et

$R^3$ représente un atome d'hydrogène, un groupe alkyle ayant 1 à 18 atomes de carbone, un groupe cycloalkyle ayant 5 à 14 atomes de carbone, un groupe aralkyle ayant 7 à 16 atomes de carbone ou un groupe aryle ayant 6 à 20 atomes de carbone, et/ou une amidine cyclique de formule (II)

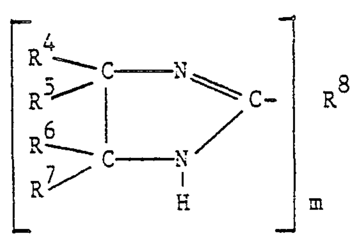

(II)

dans laquelle :

$R^4$, $R^5$, $R^6$, $R^7$ représentent chacun un atome d'hydrogène, un reste alkyle ayant 1 à 6 atomes de carbone, un reste cycloalkyle ayant 5 à 10 atomes de carbone, un reste aralkyle ayant 7 à 12 atomes de

carbone, un reste aryle ayant 6 à 15 atomes de carbone ou un reste hétérocyclique ayant 5 à 10 atomes de carbone et 1 ou 2 atomes d'oxygène, d'azote et/ou de soufre, et

$R^8$ représente un reste alkyle ayant 1 à 12 atomes de carbone, un reste alkylène ayant 1 à 6 atomes de carbone, un reste aryle ou alkylène ayant 6 à 15 atomes de carbone, les restes ci-dessus pouvant être substitués chaque fois par au moins un reste alkyle en $C_1$-$C_6$, cycloalkyle en $C_5$-$C_{10}$, aralkyle en $C_7$-$C_{12}$, aryle en $C_6$-$C_{15}$ ou au moins un reste hétérocyclique ayant 5 à 10 atomes de carbone et comportant 1 ou 2 atomes d'oxygène, d'azote et/ou de soufre, la substitution du reste alkyle en $C_1$-$C_{12}$ par un reste alkyle en $C_1$-$C_6$ étant exclue,

m vaut 1 ou 2, et

si m vaut 1, $R^8$ peut représenter aussi un atome d'hydrogène.